# EUROPEAN PATENT APPLICATION

(11) **EP 2 022 425 A2**
(43) Date of publication of application: **11.02.2009**
(21) Application number: 08161595.7
(22) Date of filing: 31.07.2008
(51) Int. Cl.: A61B 17/70, A61B 17/00, A61F 2/44, A61B 17/80

(54) **Dynamic extension plate for anterior cervical fusion**

(30) Priority: 09.08.2007 US 836439
(71) Applicant: Aesculap AG, 78532 Tuttlingen (DE)
(72) Inventor: Tyber, Jeffrey, Bethlehem, PA 18015 (US); Wing, Charles, Center Valley, PA 18034 (US); Halleck, Robert, Lake Hopatcong, NJ 07849 (US); Cole, Jeff, Macungie, PA 18062 (US)
(74) Representative: Hoeger, Stellrecht & Partner Patentanwälte

(57) **Abstract**

An osteosynthetic plate assembly and a method of installing the osteosynthetic plate assembly is disclosed. The osteosynthetic plate assembly comprises a dynamic extension plate having a first end portion for connection to a first vertebrae, a second end portion for connection to a second vertebrae, and a dynamic flexible portion extending between the first and second end portions. The dynamic extension plate is configured for coupling with a cervical fusion plate. A method of installing the dynamic extension plate to adjacent vertebrae comprises the steps of engaging a connector of the dynamic extension plate with a coupling of a cervical fusion plate, and mounting the dynamic extension plate to the adjacent vertebrae.

## Description

### FIELD OF THE INVENTION

The present invention relates generally to controlling kinematics of the body, and more specifically to a dynamic extension for an implant, and a method for installing a dynamic extension to an implant to control the kinematics of areas adjacent to the implant.

### BACKGROUND OF THE INVENTION

Anterior cervical discectomy and fusion (ACDF) is a common surgical procedure for treating nerve root or spinal cord compression caused by a degenerated or herniated disc. In the course of an ACDF procedure, the surgeon enters the intervertebral space and removes all or a portion of a degenerated disc. The intervertebral space is then filled with bone graft. The graft is intended to accelerate the biological fusion of adjacent vertebrae. The bone graft may be an autograft, allograft, synthetic bone substitute, or bone morphogenic protein (BMP). Alternatively, a spacer having a hollow center that is pre-filled with bone graft may be positioned in the intervertebral space.

The process of joining the vertebrae together with bone graft is commonly referred to as "fusion." In many instances, a bone fixation plate (referred to herein as an osteosynthetic plate or plating system) is fastened to the vertebrae directly adjacent the fusion site with bone fasteners. The bone fixation plate stabilizes the vertebrae directly adjacent the fusion site to promote fusion of those vertebrae.

While the fusion may alleviate the pain in the fusion site, complications can develop. When vertebra are fused, the spine loses mobility at the fused location. As a result, vertebral discs that are in proximity to the fused location must make up for the lost mobility. In many cases, neighboring discs must provide a wider range of motion and withstand larger stresses than prior to the fusion. The added stress on a neighboring disc can lead to accelerated degeneration of the disc, causing additional pain and suffering for the patient. This can lead to further revision surgery to fuse more vertebrae adjacent to the original fusion site.

In the course of a common revision surgery, the previously implanted osteosynthetic plate is removed and replaced with a longer plate such that the fused vertebrae and the vertebrae adjacent to the fused vertebrae can be immobilized together by a single plate. To remove the previously implanted osteosynthetic plate, each bone fastener must be removed, some of which may be overgrown with bone. Explanting the old bone fasteners and implanting new bone fasteners is a highly invasive, risky procedure. In view of the foregoing, a need exists for an improved osteosynthetic plating system with improved interconnectivity such that revision surgery is less invasive and traumatic for the patient. In addition, a need exists to slow or even prevent the deterioration of discs that neighbor the fusion site, so as to reduce the need for revision surgery.

### SUMMARY OF THE INVENTION

According to one aspect of the invention, an osteosynthetic plate assembly comprising a dynamic extension plate is provided. The dynamic extension plate includes a first end portion for connection to a first vertebrae, a second end portion for connection to a second vertebrae, and a dynamic flexible portion extending between the first and second end portions. The first end portion or second end portion comprises a connector for coupling to an adjacent plate component.

According to another aspect of the invention, an osteosynthetic plate assembly comprises a dynamic extension plate and a cervical fusion plate configured for connection to one of the first and the second end portions of the dynamic extension plate.

According to yet another aspect of the invention, an osteosynthetic plate assembly comprises a dynamic extension plate having a dynamic flexible portion extending between the first and second end portions. The dynamic flexible portion comprises at least one elongated flexible member for limiting relative motion of the first and second vertebrae.

Moreover, a spinal surgical method is described. The method comprises the step of engaging a connector of a dynamic extension plate with a coupling of a cervical fusion plate. The dynamic extension plate is mounted to the adjacent vertebrae.

Furthermore, a spinal surgical method comprises the step of fastening a cervical fusion plate at a fusion site between a first vertebra and a second vertebra. A disc in proximity to the fusion site that is susceptible to accelerated disc degeneration as a result of the fusion site is identified, and the limited range of mobility of that disc is determined. A dynamic extension plate that provides the limited range of mobility for the disc is selected. The selected dynamic extension plate is implanted over the disc.

Moreover, a spinal surgical method comprises the step of fastening a first plate between a first vertebra and a second vertebra, wherein the first plate provides resistance to motion in a plane relative to the spine. A second plate is fastened to the first plate, wherein the second plate spans between the second vertebra and a third vertebra adjacent to the second vertebra. The second plate provides less resistance to motion in the plane relative to the spine than the first plate.

According to still another aspect of the invention, an osteosynthetic plate assembly includes a spinal implant and a dynamic extension extending from the spinal implant, the spinal implant and dynamic extension forming a single one-piece body of unitary construction.

According to still another aspect of the invention, an assembly includes an interbody implant and a dynamic extension extending from the interbody implant. In one embodiment, the interbody implant is an intervertebral disc prosthesis.

### PREFERRED EMBODIMENTS OF THE INVENTION

A first preferred embodiment of the invention relates to an osteosynthetic plate assembly comprising a dynamic extension plate, the dynamic extension plate having a first end portion for connection to a first vertebrae, a second end portion for connection to a second vertebrae, and a dynamic flexible portion extending between the first and second end portions, the first end portion or second end portion comprising a connector for coupling to an adjacent plate component.

Preferably, the dynamic flexible portion comprises at least one elongated flexible member extending along a longitudinal axis of the osteosynthetic plate assembly for limiting relative motion of the first and second vertebrae.

Advantageously, a thickness dimension of the elongated flexible member is less than a thickness dimension of either the first end portion or the second end portion.

Preferably, the osteosynthetic plate assembly further comprises at least one aperture formed within the elongated flexible member.

Advantageously, the at least one aperture has a substantially elliptical shape for facilitating deflection of the elongated flexible member along the longitudinal axis.

Preferably, the osteosynthetic plate assembly further comprises a plurality of apertures formed within the elongated flexible member, each aperture having a substantially elliptical shape.

Advantageously, the dynamic flexible portion comprises at least two elongated flexible members that extend along a longitudinal axis of the plate assembly for limiting relative motion of the first and second vertebrae.

Preferably, the osteosynthetic plate assembly of claim 7, wherein the at least two elongated flexible members extend laterally toward the longitudinal axis of the plate assembly.

Advantageously, the connector comprises a flexible tab.

Preferably, the connector comprises a channel.

Advantageously, the osteosynthetic plate assembly further comprises a cervical fusion plate.

Preferably, the cervical fusion plate comprises a coupling for mating with the connector.

Advantageously, the modulus of elasticity of the dynamic flexible portion is between about 10 kPa to about 200 GPa.

Preferably, the dynamic flexible portion comprises at least one tension-applying member for separating the first and second vertebrae along the longitudinal axis.

A further preferred embodiment of the invention relates to an osteosynthetic plate assembly comprising:
a dynamic extension plate having a first end portion for connection to a first vertebrae, a second end portion for connection to a second vertebrae, and a dynamic flexible portion extending between the first and second end portions; and
a cervical fusion plate configured for connection to one of the first and the second end portions of the dynamic extension plate.

Advantageously, the dynamic extension plate comprises a connector and the cervical fusion plate comprises a coupling for mating with the connector.

Preferably, the connector comprises one of a flexible tab and a channel, and the coupling comprises the other of said flexible tab and said channel, wherein the flexible tab has a geometry that conforms to the geometry of the channel.

Advantageously, the dynamic flexible portion of the dynamic extension plate comprises at least one tension-applying member for separating the first and second vertebrae along the longitudinal axis.

Preferably, the dynamic flexible portion comprises at least one elongated flexible member for limiting relative motion of the first and second vertebrae.

Advantageously, the osteosynthetic plate assembly further comprises another cervical fusion plate configured for connection to the other of the first and the second end portions of the dynamic extension plate.

Preferably, the osteosynthetic plate assembly further comprises another dynamic extension plate configured for connection to the cervical fusion plate, wherein the cervical fusion plate is configured for mounting two dynamic extension plates.

A further preferred embodiment of the invention relates to an osteosynthetic plate assembly comprising a dynamic extension plate, the dynamic extension plate having a first end portion for connection to a first vertebrae, a second end portion for connection to a second vertebrae, and a dynamic flexible portion extending between the first and second end portions, the dynamic flexible portion comprising at least one elongated flexible member for limiting relative motion of the first and second vertebrae.

Moreover, a spinal surgical method is described, the method comprising the steps of:
engaging a connector of a dynamic extension plate with a coupling of a cervical fusion plate; and
mounting the dynamic extension plate to the adjacent vertebrae.

Advantageously, the method further comprises the step of fastening the cervical fusion plate to one of the adjacent vertebrae.

Preferably, the engaging step comprises the sub-steps of inserting a tab portion of the connector into a channel of the coupling of the cervical fusion plate, rotating the tab portion within the channel, and seating the tab portion in the channel.

Advantageously, the cervical fusion plate is implanted.

Preferably, the method comprises the step of compressing the dynamic extension plate prior to the step of mounting the dynamic extension plate.

Moreover, a spinal surgical method is described, the method comprising the steps of:
fastening a cervical fusion plate at a fusion site between a first vertebra and a second vertebra;
identifying a disc in proximity to the fusion site that is susceptible to accelerated disc degeneration as a result of the fusion site;
determining a limited range of mobility for the disc;
selecting a dynamic extension plate that provides the limited range of mobility for the disc; and
implanting the dynamic extension plate over the disc.

Preferably, the disc lies adjacent to the fusion site.

Moreover, a spinal surgical method is described, the method comprising the steps of:
fastening a first plate between a first vertebra and a second vertebra, the first plate providing resistance to motion in a plane relative to the spine; and
fastening a second plate to the first plate, the second plate spanning between the second vertebra and a third vertebra adjacent to the second vertebra, the second plate providing less resistance to motion in said plane relative to the spine than the first plate.

Preferably, the first plate is a cervical fusion plate.

Advantageously, the second plate is a dynamic extension plate.

Preferably, motion in said plane is one of torsion, lateral motion, flexion, extension, compression and expansion.

Advantageously, the dynamic extension plate described above is formed of one of a shape-memory alloy and a shape-memory polymer.

A further preferred embodiment of the invention relates to an osteosynthetic plate assembly comprising a spinal implant and a dynamic extension extending from the spinal implant, the spinal implant and dynamic extension forming a single one-piece body of unitary construction.

Preferably, the spinal implant comprises a fusion plate.

Advantageously, the spinal implant comprises an intervertebral disc prosthesis.

### BRIEF DESCRIPTION OF THE DRAWINGS

The invention is best understood from the following detailed description when read in connection with the accompanying drawings, with like elements having the same reference numerals. Included in the drawings are the following figures:
FIG. 1 depicts an exemplary embodiment of an osteosynthetic plate assembly mounted to a vertebral column according to one aspect of the invention;
FIG. 2 depicts an exploded perspective view of the osteosynthetic plate assembly illustrated in FIG. 1;
FIG. 3 depicts a perspective view of the dynamic extension plate illustrated in FIG. 2;
FIG. 4 is an enlarged plan view of end segments of the osteosynthetic plate assembly of FIG. 2;
FIG. 5A is a cross sectional view of the end segments of the osteosynthetic plate assembly of FIG. 4 taken along the lines 5-5, whereby the mating segments of the osteosynthetic plate assembly are illustrated in a pre-mated configuration;
FIG. 5B illustrates the mating segments of the osteosynthetic plate assembly of FIG. 5A in a mated configuration;
FIGS. 6A-6G depict perspective views of alternative exemplary embodiments of a dynamic extension plate according to aspects of the invention;
FIG. 7 depicts a perspective view of another exemplary configuration of an osteosynthetic plate assembly in accordance with the invention, comprising a cervical fusion plate mounted between two dynamic extension plates;
FIG. 8 depicts an exemplary embodiment of yet another configuration of an osteosynthetic plate assembly mounted to a vertebral column, wherein the osteosynthetic plate assembly comprises a dynamic extension plate mounted between two cervical fusion plates;
FIG. 9 depicts a perspective view of the dynamic extension plate illustrated in FIG. 8;
FIG. 10 depicts a free body diagram of the right side of the osteosynthetic plate assembly and vertebral column segment of FIG. 1;
FIG. 11 is a perspective view of an exemplary embodiment of another configuration of an osteosynthetic plate assembly; and
FIG. 12 is an exemplary embodiment of yet another assembly in accordance with the present invention, wherein the assembly is mounted to a vertebral column, and includes an interbody implant with dynamic extensions.

### DETAILED DESCRIPTION OF THE INVENTION

Although the invention is illustrated and described herein with reference to specific embodiments, the invention is not intended to be limited to the details shown. Rather, various modifications may be made in the details within the scope and range of equivalents of the claims and without departing from the invention.

The problems associated with spinal surgery and revision procedures are resolved in several respects by osteosynthetic plate assemblies and procedures for using the assemblies, all in accordance with the present invention. The applicants generally propose to protect discs that lie in proximity to fusion sites ("neighboring discs") from accelerated degeneration by controlling the kinematics of those discs. The mobility of the neighboring discs is controlled without resorting to further fusion, which would sacrifice all mobility at those disc sites. Controlling the kinematics of neighboring discs protects the neighboring discs from damage caused by increased stress, while still preserving some mobility at those sites. A preferred way of controlling kinematics of neighboring discs in accordance with the invention is to provide a gradual change in mobility between the fused site and adjacent sites, rather than an abrupt change between the fused site and adjacent disc spaces.

In one exemplary embodiment of this procedure, a neighboring disc that appears susceptible to accelerated degeneration is identified, and an appropriate range of motion for that disc that does not carry a risk of accelerated degeneration is selected. A dynamic restraint that provides the desired range of motion is then implanted at the site of the neighboring disc. The dynamic restraint limits the mobility of the neighboring disc so that the disc does not become susceptible to accelerated degeneration. This, in turn, reduces the potential for pain and discomfort in the neighboring disc, thereby avoiding further revision surgery. The procedure for adding a dynamic restraint at a vertebral disc adjacent or in proximity to a fused site may be referred to as "topping off."

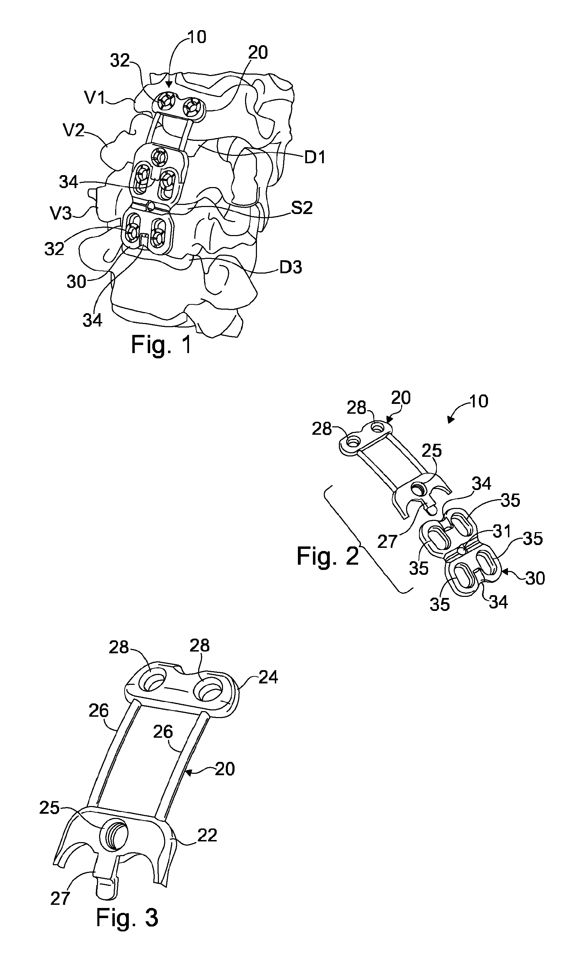

A number of osteosynthetic plate assemblies are contemplated in accordance with the present invention. FIG. 1 depicts an exemplary embodiment of an osteosynthetic plate assembly 10 mounted to a vertebral column according to one aspect of the invention. The osteosynthetic plate assembly 10 comprises a cervical fusion plate 30 coupled to a dynamic extension plate 20. The cervical fusion plate 30 (hereinafter plate 30) is fixedly mounted to the anterior side of vertebrae V2 and V3 by four threaded bone fasteners 32, and the dynamic extension plate 20 (hereinafter plate 20) is mounted to the anterior side of vertebrae V1 and V2 by three threaded bone fasteners 32. Vertebrae V1-V3 may be cervical vertebrae of a human spine, for example, or any other particular vertebrae of a vertebral column.

The cervical fusion plate 30 stabilizes vertebrae V2 and V3 to promote fusion of those vertebrae with spacer S2. While the fusion of V2 and V3 may alleviate the pain at the fusion spacer site S2, pressures along the spine are transferred onto one or more adjacent discs, such as D1, which may cause rapid deterioration those discs, and additional pain and suffering for the patient. For those reasons, dynamic extension plate 20 is mounted to plate 30 and vertebrae V1 and V2 to limit or prevent degeneration of the neighboring discs, such as D1.

Cervical fusion plate 30 is sufficiently rigid to prevent relative motion of vertebrae V2 and V3. In contrast, dynamic extension plate 20 is flexible to permit limited and controlled relative movement of vertebrae V1 and V2. By limiting relative motion of the adjacent vertebrae V1 and V2, the dynamic extension plate 20 limits stresses exerted on disc D1 and therefore slows or prevents degeneration of disc D1. The plate 20 is uniquely adapted to absorb pressure at disc D1 and re-distribute that pressure across a larger segment of the spine thereby limiting stress concentrations at one particular disc.

According to one exemplary use of the invention, the cervical fusion plate 30 is mounted to vertebrae V2 and V3 for fusing those vertebrae in an initial fusion surgery. In a follow-up revision surgery performed at some time after the initial fusion surgery, the dynamic extension plate 20 is mounted to plate 30 and adjacent vertebrae V1 and V2 to improve or prolong the condition of disc D1. The time span between initial surgery and the revision surgery may vary.

In the revision surgery, dynamic extension plate 20 may be coupled to plate 30 and fastened to V1 and V2 while plate 30 remains implanted in vertebrae V2 and V3, by virtue of the unique design of osteosynthetic plate assembly 10. Therefore, removal of the implanted plate 30 from vertebrae V2 and V3 is not required in the revision surgery. Accordingly, the disadvantages associated with the removal of a previously implanted cervical fusion plate are avoided, as described in the Background section.

According to another exemplary use of the invention, the entire osteosynthetic plate assembly 10 may be installed in the initial surgery for preventative measures. The plates 20 and 30 may be coupled together before or after plate 30 is implanted into the spine.

The manufacturing cost of a dynamic extension plate 20 is lower than the manufacturing cost of a large replacement plate for fusing V1, V2 and V3. The dynamic extension plate 20 is particularly advantageous in that it may be mounted to an implanted fully-constrained, semi-constrained, semi dynamic or fully dynamic cervical plate and fastened to the neighboring vertebrae.

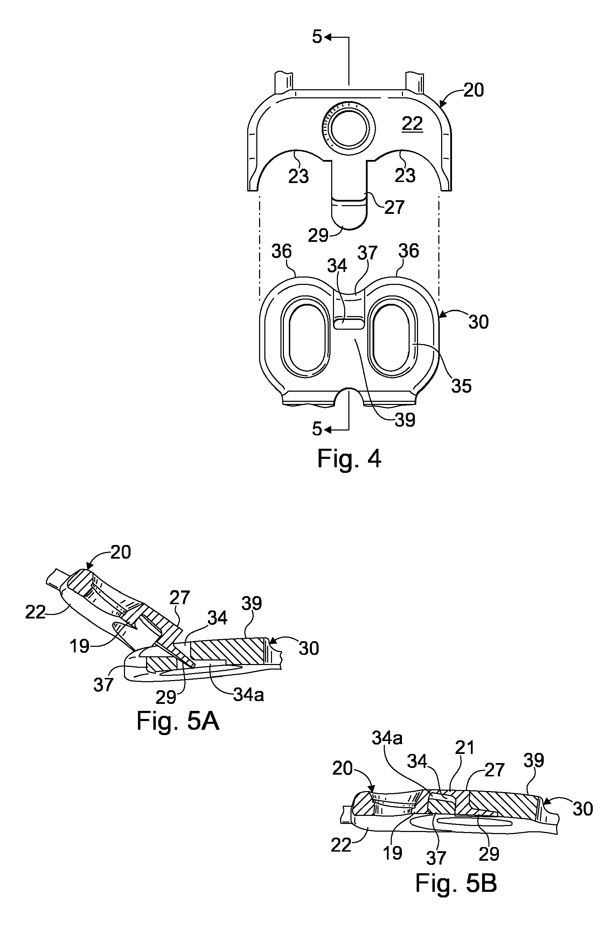

FIG. 2 depicts an exploded perspective view of the osteosynthetic plate assembly 10 illustrated in FIG. 1. The cervical fusion plate 30 is a substantially rectangular rigid body comprising four slots 35 for carrying threaded bone fasteners 32 (see FIG. 1). A window 31, in the form of a circular opening, is provided in the central region of plate 30 for viewing the intervertebral space. The window 31 is a convenient feature for assessing the progress of the fusion site, or readjusting the position of a fusion spacer, such as fusion spacer S2, subsequent to mounting spacer S2 in the intervertebral space. Fusion plates in accordance with the invention may include one or more couplings on each end thereof for cooperative engagement with dynamic extension plates or other fusion plates. In FIGS. 1 and 2, the fusion plate 30 includes two couplings 34. One of the couplings 34 receives a mating connector of the dynamic extension plate 20, as best described with references to FIGS. 4, 5A and 5B.

FIG. 3 depicts another perspective view of the dynamic extension plate 20. The plate 20 generally comprises an end portion 24 for mounting to a vertebrae (such as V1), an opposing end portion 22 for mounting to a vertebrae (such as V2), and a pair of flexible members 26 extending between the end portions 22 and 24. The end portion 22 includes an aperture 25 for receiving a threaded fastener (see FIG. 1) for rigid connection to a vertebrae, and a connector 27 extending from an end thereof for releasably mating with one of the couplings 34 of fusion plate 30. The end portion 24 includes two apertures 28, each for receiving a threaded fastener (see FIG. 1) for rigid connection to a vertebrae.

The pair of flexible members 26 extend in a parallel arrangement between the end portions 22 and 24 of plate 20. The flexible members 26 are generally referred to herein as the 'dynamic flexible portion' of extension plate 20. The flexible members 26 of plate 20 are adapted to permit limited relative motion of adjacent vertebrae V1 and V2 along or about various axes of the spinal cord, while restricting relative motion of adjacent vertebrae V1 and V2 along or about other axes.

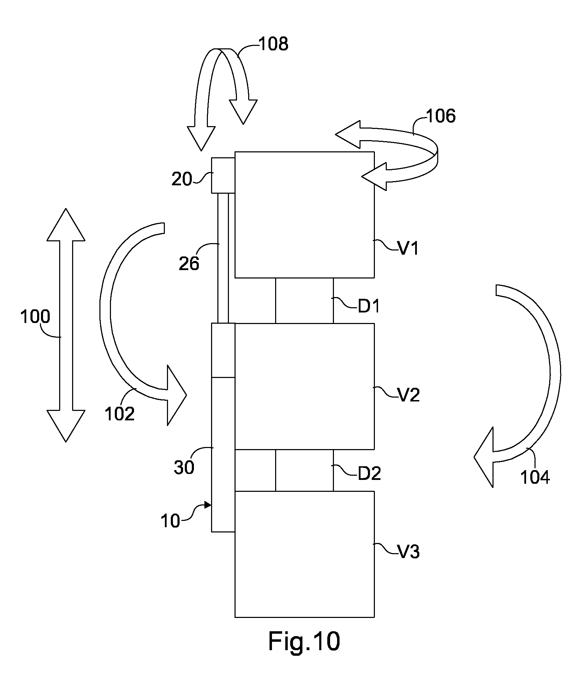

More specifically, and referring to the free body diagram illustrated in FIG. 10, the flexible members 26 are uniquely adapted to resist torsion (arrows 106), lateral motion (arrows 108), compression and expansion (arrows 100), while permitting a limited range of flexion (arrow 102) and extension (arrow 104) of adjacent vertebrae V1 and V2, relative to each other. The range of motion is limited by the shape, size, thickness, spatial arrangement and material composition of the flexible members 26.

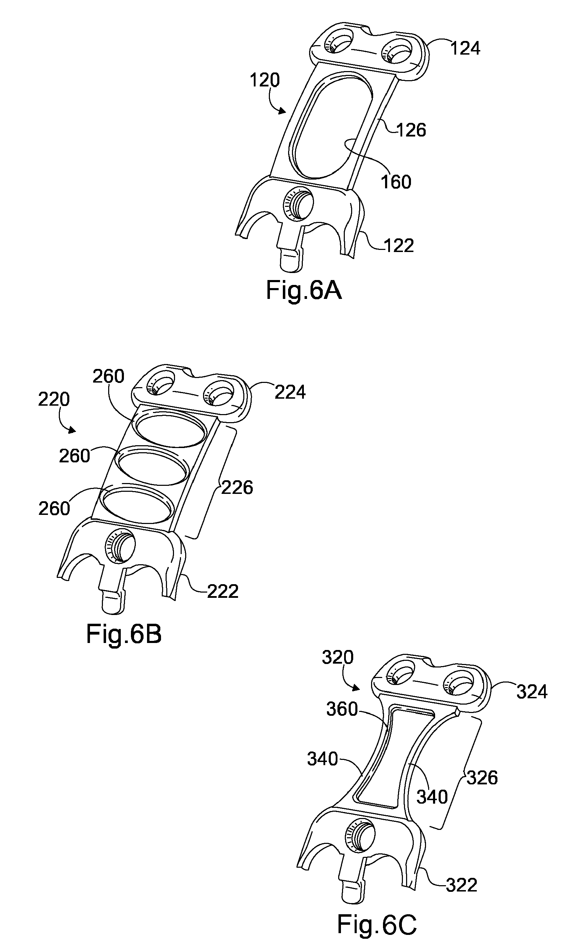

The plate 20 may include any number of flexible members 26 having any shape, size, spatial arrangement and material composition, as best shown and described with reference to the alternative embodiments of FIGS. 6A-6G, to achieve any desired kinematic effect. For example, a plate may only include a single flexible member 26 for resisting lateral motion (arrows 108), compression and expansion (arrows 100), while permitting a limited range of flexion (arrow 102), extension (arrow 104) and torsion (arrows 106) of adjacent vertebrae V1 and V2, relative to each other. According to another alternative embodiment, the plate may include two flexible members oriented in an "X" shape.

FIG. 4 depicts a detailed view of the osteosynthetic plate assembly 10 of FIG. 2. The end portion 22 of plate 20 includes two recesses 23 disposed adjacent the connector 27 for receiving lobe portions 36 of plate 30 in assembly. Recesses 23 each have a rounded curvature and are symmetrical to one another. The curvatures of the recesses 23 conform to rounded curvatures on lobes 36. In this arrangement, the contours of the lobes 36 precisely match the contours of the recesses to enhance stability and securely connect plates 20 and 30. The connector 27 comprises a flexible tab 29 extending from the body of end portion 22, and a heel 19 proximal to the body of end portion 22 (see FIGS. 5A and 5B).

The plate 30 includes two couplings 34 that include "Z"-shaped channels 34a (one shown in FIG. 4) defined on opposing sides of plate 30. Each "Z"-shaped channel 34a is configured for receiving flexible tab 29 of plate 20, and has a geometry that conforms to the geometry of the flexible tab 29. The "Z"-shaped channels 34a are each defined between a thin section 37 and a thick central portion 39 of plate 20, as best illustrated in FIGS. 5A and 5B.

FIGS. 5A and 5B depict cross sectional views of a portion of osteosynthetic plate assembly 10 of FIG. 4 taken along the lines 5-5 in different mating configurations. More specifically, plates 20 and 30 are illustrated in a pre-mated configuration in FIG. 5A, and plates 20 and 30 are illustrated in a mated configuration in FIG. 5B.

According to one exemplary method of assembling plates 20 and 30, flexible tab 29 of connector 27 is inserted into coupling 34 of plate 30 from the side of thin section 37 at an oblique angle, as best shown in FIG. 5A. In the course of inserting tab 29 into coupling 34, plate 20 is rotated downwardly, or toward the plane of plate 30, causing thin section 37 and/or tab 29 to deflect slightly until tab 29 is seated beneath central section 39, and the curved surface of heel 19 is "snapped" over the rounded edge of thin section 37 of plate 20, as best shown in FIG. 5B. The interference fit between connector 27 and coupling 34 limits detachment of plates 20 and 30.

By virtue of the geometry of flexible tab 29 and "Z"-shaped channel 34a of coupling 34, plate 20 may be assembled with plate 30 regardless of whether plate 30 is already mounted to the spine. The connection between tab 29 and coupling 34 does not require the use of separate screws or fasteners, and can be made while plate 30 is mounted to the spine, with plate 30 detached from the spine or while the plate is coupled with other plates. The tab 29 is easily snap-fit into coupling 34 by virtue of the positive engagement between heel 19 and thin section 37.

FIGS. 6A-6G depict perspective views of alternative dynamic extension plates 120, 220, 320, 620, 720, 820 and 920 according to aspects of the invention. The dynamic flexible portion of a dynamic extension plate may be tailored to suit the unique needs of a patient, as described with reference to those alternative embodiments.

FIG. 6A depicts a plate 120 including end portions 122 and 124, and a rectangular dynamic flexible portion 126 extending therebetween. The flexible portion 126 includes an aperture 160 formed therein. Depending upon its material composition, flexible portion 126 may be configured to resist torsion (arrows 106 of FIG. 10) and lateral motion (arrows 108), while permitting a limited range of flexion (arrow 102), extension (arrow 104), compression and expansion (arrows 100) of adjacent vertebrae.

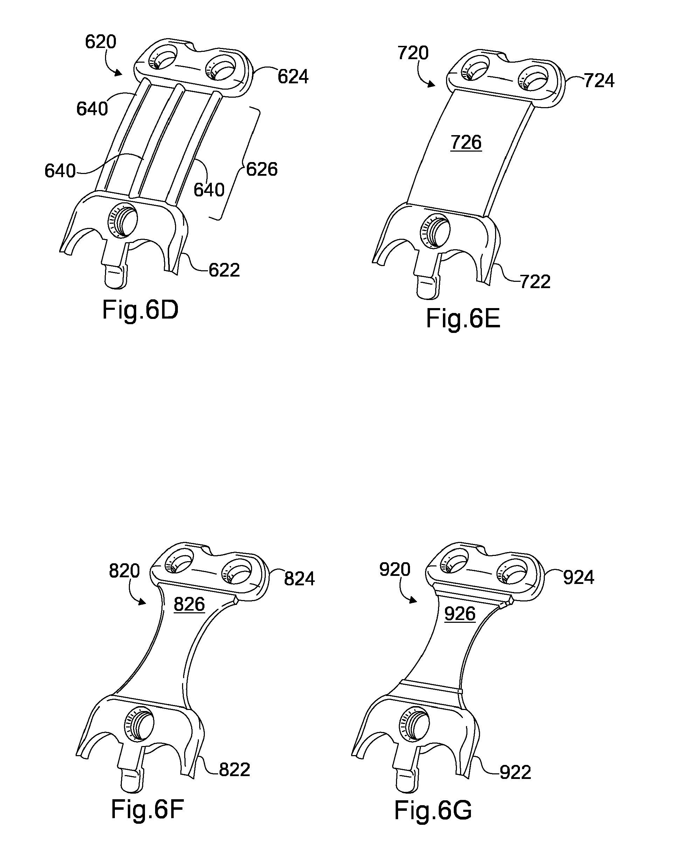

The individual features of plate 120 may be modified to evoke a specific mechanical response. For example, the thickness dimension of the flexible portion 126 may be maintained less than a thickness dimension of end portions 122 and 124, as shown, for a greater range of flexion (arrow 102) and extension (arrow 104). Additionally, the size of aperture 160 may be increased for a greater range of compression and expansion (arrows 100). According to another alternative embodiment shown in FIG. 6E, the flexible portion is a solid plate and does not include an aperture. Generally, adding material to flexible portion 126 increases the rigidity of plate 120, consequently increasing its resistance to torsion (arrows 106 of FIG. 10), lateral motion (arrows 108), compression and expansion (arrows 100), flexion (arrow 102) and extension (arrow 104).

FIG. 6B depicts a plate 220 including end portions 222 and 224, and a rectangular dynamic flexible portion 226 extending therebetween. The flexible portion 226 incorporates three elliptical apertures 260. By virtue of the shape of apertures 260 and the material composition of plate 220, the flexible portion 226 is compressible and expandable in the longitudinal direction of the spine (i.e., along arrows 100). Specifically, the elliptical apertures 260 are designed to allow the plate 220 to stretch or elongate under expansion force, and to contract under compression force. Furthermore, depending upon its material composition, the flexible portion 226 may be configured to resist torsion (arrows 106) and lateral motion (arrows 108), while permitting a limited range of flexion (arrow 102) and extension (arrow 104) of adjacent vertebrae. The material of plate 220 is resilient so that the plate can return to its original configuration after being expanded, compressed, or otherwise deformed.

According to one exemplary use of plate 220, the plate 220 may be implanted in adjacent vertebrae in a compressed state. Once implanted, the pre-compressed flexible portion 226 expands to its original shape and urges the adjacent vertebrae apart, thereby decompressing the disc between the separated vertebrae.

The plate 220 may include any number of apertures 260 having any desired shape or size. Moreover, apertures 260 may be oriented in any desired direction for tailoring the direction of the force of expansion.

FIG. 6C depicts a plate 320 including end portions 322 and 324, and a dynamic flexible portion 326 extending therebetween. The flexible portion 326 includes two flexible members 340 and an aperture 360 formed therebetween. The flexible members 340 are designed to deflect in a lateral direction (toward or away from each other) under the force of compression or expansion and lateral motion (arrows 108). For example, the flexible members 340 move apart in a lateral direction under the force of compression, and converge together in the lateral direction under the force of expansion. Lateral deflection of flexible members 340 provides an advantage over anterior/posterior deflection in that the flexible portion 326 will not contact any soft tissue anterior to the spine while in a deflected state.

In sum, depending upon its material composition, the flexible portion 326 may be configured to resist or even prevent torsion (arrows 106), while permitting a limited range of flexion (arrows 102), compression and expansion (arrows 100), lateral motion (arrows 108) and extension (arrows 104) of adjacent vertebrae. Similar to the exemplary use of plate 220, the plate 320 may be implanted in adjacent vertebrae in a compressed state for decompressing the disc in the intervertebral space.

FIG. 6D depicts a plate 620 including end portions 622 and 624, and a dynamic flexible portion 626 extending therebetween. The flexible portion 626 includes three flexible members 640 oriented in parallel. The flexible portion 626 is designed to resist torsion (arrows 106), lateral motion (arrows 108), compression and expansion (arrows 100), while permitting a limited range of flexion (arrow 102) and extension (arrow 104) of adjacent vertebrae V1 and V2, relative to each other. As stated previously, the flexible portion may include any number of flexible members to evoke a specific kinematic response.

FIG. 6E depicts a plate 720 including end portions 722 and 724, and a dynamic flexible portion 726 extending therebetween. The flexible portion 726 is similar to flexible portion 126 of FIG. 6A, however aperture 160 is omitted from plate 720. The flexible portion 726 may be configured to resist torsion (arrows 106 of FIG. 10) and lateral motion (arrows 108), while permitting a limited range of flexion (arrow 102), extension (arrow 104), compression and expansion (arrows 100) of adjacent vertebrae.

FIG. 6F depicts a plate 820 including end portions 822 and 824, and a dynamic flexible portion 826 extending therebetween. The flexible portion 826 is similar to flexible portion 326 of FIG. 6C, with the exception that flexible portion 826 does not include an aperture 360. The flexible portion 826 may be configured to resist torsion (arrows 106 of FIG. 10), compression and expansion (arrows 100), while permitting a limited range of flexion (arrow 102), lateral motion (arrows 108) and extension (arrow 104) of adjacent vertebrae.

FIG. 6G depicts a plate 920 including end portions 922 and 924, and a dynamic flexible portion 926 extending therebetween. The thickness dimension of dynamic flexible portion 926 progressively decreases toward its central portion. Similar to dynamic flexible portion 826 of FIG. 6F, flexible portion 926 may be configured to resist torsion (arrows 106 of FIG. 10), compression and expansion (arrows 100), while permitting a limited range of flexion (arrow 102), lateral motion (arrows 108) and extension (arrow 104) of adjacent vertebrae. Depending upon its material composition, dynamic flexible portion 926 is adapted to permit a greater range of flexion (arrow 102) and extension (arrow 104) of adjacent vertebrae as compared with flexible portion 826 of FIG. 6F, by virtue of the thin central portion of flexible portion 926.

The flexible portion of dynamic extension plates 120, 220, 320, 620, 720, 820 and 920 is preferably formed from an elastic material that is configured to return to its original shape. By way of non-limiting example, the flexible portion of the plates 120, 220, 320, 620, 720, 820 and 920 may be formed from spring stainless steel, titanium, nickel titanium, or any other bio-compatible metallic material, or combination thereof, which undergoes stress induced martensitic phase transformation with a portion of recoverable strain. The flexible portion may also be formed from a bio-compatible synthetic material having a glass transition temperature below both body temperature (∼37 °C (98 °F)) and room temperature (∼21 °C (70 °F)), or any other bio-compatible material in its rubbery and transition state, as defined by a thermal-mechanical curve. All or a portion of the dynamic extension plates 20, 120, 200 and 320 may be formed from any of the aforementioned materials. The material that is selected may have a broad range of physical properties, the selection of which may depend on factors including but not limited to the desired amount of flexibility of the flexible portion. Preferably, the material has a modulus of elasticity of between about 10 kPa to about 200 GPa. In addition, the material preferably has a yield strength of between about 4 kPa and about 1200 MPa. Materials having properties outside of these ranges may also provide excellent performance, however.

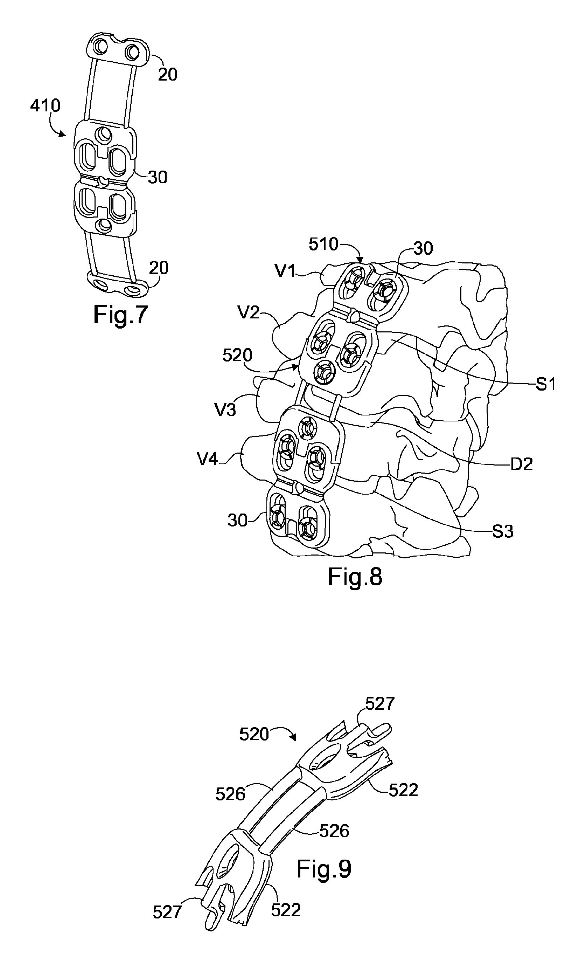

FIG. 7 depicts a perspective view of another configuration of an osteosynthetic plate assembly 410 comprising a cervical fusion plate 30 mounted between two dynamic extension plates 20. The plate assembly 410 is similar to plate assembly 10 of FIG. 1, with the exception that plate assembly 410 includes an additional dynamic extension plate 20. As best shown in FIG. 2, plate 30 includes two opposing couplings 34, each configured for receiving a connector 27 of a plate 20, thereby being capable of receiving two dynamic extension plates 20.

A surgeon may opt to implant plate assembly 410 to suspend the deterioration of two discs (D1 and D3 of FIG. 1, for example) neighboring the fusion site (spacer S2). Alternatively, an additional dynamic extension plate 20 may be implanted in a revision surgery.

FIG. 8 depicts an exemplary embodiment of another osteosynthetic plate assembly 510 mounted to the anterior side of a vertebral column according to another aspect of the invention. In this embodiment, plate assembly 510 comprises a single dynamic extension plate 520 coupled between two cervical fusion plates 30. The plates 520 and 30 are fixedly mounted to the anterior side of vertebrae V1-V4 by ten threaded bone fasteners, as shown.

The plate assembly 510 is adapted for promoting fusion at fusion spacer sites S1 and S3 and preventing stress concentrations on neighboring disc D2. It should be understood that vertebrae V1 and V2, and vertebrae V3 and V4 are fused together in this embodiment. Vertebrae V2 and V3 are not fused together.

While the fusion of vertebrae V1/V2 and vertebrae V3/V4 may alleviate pain at fusion spacer sites S1 and S3, pressures along the spine may be transferred onto disc D2 causing rapid degeneration of disc D2 in the absence of dynamic extension plate 520. The plate 520 is uniquely adapted to absorb pressure at disc D2 and re-distribute that pressure across a larger segment of the spine thereby limiting stress concentrations at disc D2.

According to one exemplary use of the plate assembly 510, the entire assembly 510 may be implanted in a single surgical procedure. Alternatively, plate 520 and a single cervical plate 30 may be coupled to an existing implanted cervical plate 30 in a revision surgery. As another alternative, plate 520 may be coupled between two implanted cervical fusion plates 30 in a revision surgery depending upon the elasticity of the plate 520.

FIG. 9 depicts a perspective view of the dynamic extension plate 520 illustrated in FIG. 8. The dynamic extension plate 520 includes two end portions 522 and two parallel flexible members 526 extending between end portions 522. The plate 520 is similar to plate 20 of FIG. 3, but that each end portion 522 of plate 520 includes a connector 527 for coupling with a cervical fusion plate.

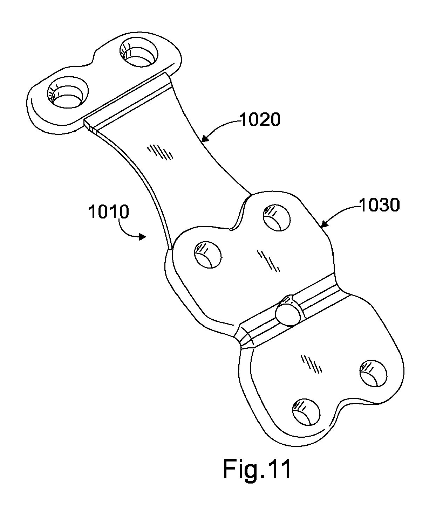

Referring now to FIG. 11, a further exemplary embodiment of an osteosynthetic plate assembly 1010 is shown in accordance with the invention is shown. Assembly 1010 is similar in certain respects to the embodiments described thus far, but integrates the dynamic topping off portion with the fusion portion in a single body having a unitary one-piece construction. In particular, assembly 1010 includes a cervical fusion portion 1030 having an integral dynamic extension 1020. This embodiment may be preferred under circumstances where dynamic restraint of discs neighboring the fusion site is contemplated at the initial implantation. As with other embodiments, fusion portion 1030 is rigid to stabilize and promote fusion of vertebrae, while dynamic extension 1020 is somewhat flexible to permit controlled relative movement of neighboring vertebrae. One or both of the fusion portion 1030 and dynamic extension 1020 may include connectors as described previously to allow modular attachment of the assembly 1010 to other plates or assemblies as described herein.

It should be understood that any of the embodiments contemplated herein, either unitary or modular, may be compatible and used with one another in an implantation or revision procedure. Moreover, it should be understood that assemblies may include a series of fusion portions and/or dynamic extensions, connected together either modularly or unitarily, with each portion having unique physical properties suited to a particular location on the spine.

Dynamic extensions in accordance with the present invention may be utilized under any circumstances where the natural kinematics of joints are altered unfavorably by implants at neighboring joints. The incorporation of dynamic extensions may be applied in the spine or other areas of the body. In addition to topping off spinal fusion plates, the dynamic extensions in accordance with the present invention may be used to top off other kinds of implants, including but not limited to interbody implants. For example, dynamic extensions in accordance with the invention may be associated with intervertebral disc implants to provide dynamic restraint to discs that neighbor the disc replacement site. Dynamic extensions may be modularly connected to the disc implant using, for example, the connector tabs described previously, or may be formed integrally with the disc implant. The physical properties of the dynamic extensions are chosen so as to adjust the kinematics of neighboring vertebrae, which may be dramatically changed after the disc prosthesis is implanted.

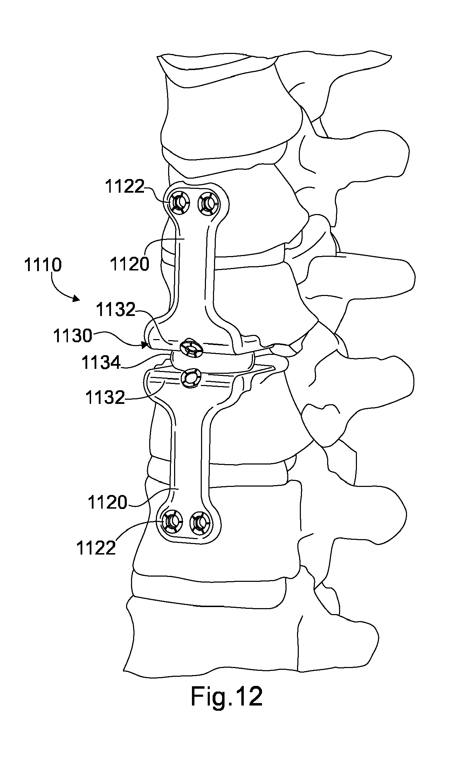

Referring now to FIG. 12, an assembly 1110 includes an intervertebral disc implant 1130 with dynamic extensions 1120 that top off the disc implant at adjacent vertebrae. The intervertebral disc implant 1130 includes a pair of end plates 1132, each end plate being secured to a vertebra. A core 1134 is positioned between the end plates 1132. Each dynamic extension 1120 is integrally formed with an end plate 1132 and extends away from the replacement disc site to an adjacent vertebra. The dynamic extensions 1120 include holes 1122 for securing the dynamic extensions to adjacent vertebrae.

While preferred embodiments of the invention have been shown and described herein, it will be understood that such embodiments are provided by way of example only. Numerous variations, changes and substitutions will occur to those skilled in the art without departing from the spirit of the invention.

For example, the cervical fusion plate may include opposing flexible tabs, while the dynamic extension plates include "Z"-shaped channels for coupling with the connectors of the cervical fusion plate. Furthermore, the osteosynthetic plate assemblies described herein are not limited to the illustrations, as an osteosynthetic plate assembly may include any number or configuration of cervical fusion and dynamic extension plates.

Accordingly, it is intended that the appended claims cover all such variations as fall within the spirit and scope of the invention.

## Claims

1. An osteosynthetic plate assembly comprising a dynamic extension plate, the dynamic extension plate having a first end portion for connection to a first vertebrae, a second end portion for connection to a second vertebrae, and a dynamic flexible portion extending between the first and second end portions, the first end portion or second end portion comprising a connector for coupling to an adjacent plate component.

2. The osteosynthetic plate assembly of claim 1, wherein the dynamic flexible portion comprises at least one elongated flexible member extending along a longitudinal axis of the osteosynthetic plate assembly for limiting relative motion of the first and second vertebrae.

3. The osteosynthetic plate assembly of claim 2, wherein a thickness dimension of the elongated flexible member is less than a thickness dimension of either the first end portion or the second end portion.

4. The osteosynthetic plate assembly of claim 2 or 3, further comprising at least one aperture formed within the at least one elongated flexible member.

5. The osteosynthetic plate assembly of claim 4, wherein the at least one aperture has a substantially elliptical shape for facilitating deflection of the at least one elongated flexible member along the longitudinal axis.

6. The osteosynthetic plate assembly according to anyone of claims 2 to 5 further comprising a plurality of apertures formed within the at least one elongated flexible member, each aperture having a substantially elliptical shape.

7. The osteosynthetic plate assembly according to anyone of the preceding claims, wherein the dynamic flexible portion comprises at least two elongated flexible members that extend along a longitudinal axis of the plate assembly for limiting relative motion of the first and second vertebrae.

8. The osteosynthetic plate assembly of claim 7, wherein the at least two elongated flexible members extend laterally toward the longitudinal axis of the plate assembly.

9. The osteosynthetic plate assembly according to anyone of the preceding claims, wherein the connector comprises a flexible tab.

10. The osteosynthetic plate assembly according to anyone of the preceding claims, wherein the connector comprises a channel.

11. The osteosynthetic plate assembly according to anyone of the preceding claims further comprising a cervical fusion plate .

12. The osteosynthetic plate assembly of claim 11, wherein the cervical fusion plate comprises a coupling for mating with the connector.

13. The osteosynthetic plate assembly according to anyone of the preceding claims, wherein the modulus of elasticity of the dynamic flexible portion is between about 10 kPa to about 200 GPa.

14. The osteosynthetic plate assembly according to anyone of the preceding claims, wherein the dynamic flexible portion comprises at least one tension-applying member for separating the first and second vertebrae along the longitudinal axis.

15. The osteosynthetic plate assembly according to anyone of the preceding claims, wherein the dynamic extension plate is formed of one of a shape-memory alloy and a shape-memory polymer.
